# EUROPEAN PATENT APPLICATION

(11) **EP 4 841 242 A1**
(43) Date of publication of application: **18.11.2026**
(21) Application number: 25890722.9
(22) Date of filing: 18.06.2025
(51) Int. Cl.: G01N 21/3563, G01N 21/359, G01N 21/27, G01N 25/04

(54) **GLASS TRANSITION TEMPERATURE TESTING METHOD AND APPARATUS, AND DEVICE, MEDIUM AND PROGRAM PRODUCT**

(30) Priority: 12.11.2024 CN 202411612384
(71) Applicant: Sinoma Wind Power Blade Co., Ltd., Beijing 100192 (CN)
(72) Inventor: WANG, Jijun, Beijing 100083 (CN); WANG, Xin, Beijing 100083 (CN)
(74) Representative: Murgitroyd & Company
(86) International application number: PCT/CN2025/101616
(87) International publication number: WO 2026/103125

(57) **Abstract**

Disclosed in the present application are a glass transition temperature testing method and apparatus, and a device, a medium and a program product. The method comprises: respectively performing near-infrared spectroscopy tests on a plurality of standard samples in a target environment under a plurality of preset conditions, so as to obtain a near-infrared spectroscopy signal of each standard sample under each preset condition; acquiring a calibrated glass transition temperature of a target polymer in each standard sample; respectively creating training samples from the near-infrared spectroscopy signal, which corresponds to each standard sample under each preset condition, and the corresponding calibrated glass transition temperature, so as to obtain a training sample set; inputting the training sample set into a glass transition temperature measurement model, so as to obtain a measured glass transition temperature corresponding to each training sample; and on the basis of the measured glass transition temperature which correspond to each training sample, and the calibrated glass transition temperature, performing iterative training, so as to obtain a trained glass transition temperature measurement model. The present application can realize rapid and accurate measurement of a glass transition temperature at an industrial production site.

## Description

### CROSS-REFERENCE TO RELATED APPLICATIONS

This application claims priority to Chinese patent application No. 202411612384.2, entitled "GLASS TRANSITION TEMPERATURE MEASURING METHOD AND APPARATUS, AND DEVICE, MEDIUM AND PROGRAM PRODUCT", filed on November 12, 2024, the entire contents of which are incorporated herein by reference.

### TECHNICAL FIELD

The present application relates to the technical field of measuring the glass transition temperature, especially to a glass transition temperature measuring method and an apparatus, a device, a computer storage medium, and a computer program product.

### BACKGROUND

The glass transition temperature (Tg) is a key parameter for characterizing the unique physical behavior of polymeric materials, which marks the critical point where the polymer chains gain sufficient energy to initiate random motion from a frozen glassy state. At this specific temperature, the material undergoes a fundamental transformation from a hard and brittle glassy state to a soft and flow-prone rubbery state (also known as a viscoelastic state), resulting in significant changes in mechanical properties such as elastic modulus and toughness. Therefore, the glass transition temperature is not only a crucial basis for determining the operational temperature range of polymeric materials but also a core indicator for evaluating material processability and product quality, which holds immense significance in the field of material science and engineering, and directly influences the product design, material selection, and the optimization and control of manufacturing processes.

Currently, the commonly used methods for measuring the glass transition temperature in industrial production are differential scanning calorimetry (DSC) and dynamic mechanical analysis (DMA). Although both DSC and DMA can provide relatively accurate results, they need to be performed in laboratory environments, which are time-consuming operations and require data analysis performed by specialized technicians. In modern factories that pursue efficient production, rapid quality inspection, and cost control, traditional measuring methods struggle to meet production demands. Consequently, there is an urgent need for an accurate method for measuring the glass transition temperature that can be used directly at industrial production sites.

### SUMMARY

The present application provides a glass transition temperature measuring method and an apparatus, a device, a computer storage medium, and a computer program product, which can rapidly and accurately measure the glass transition temperature.

In a first aspect, some embodiments of the present application provide a glass transition temperature measuring method. The method includes:
performing a near-infrared spectrum measurement on a to-be-measured object in a target environment to obtain a near-infrared spectrum signal of the to-be-measured object, the to-be-measured object containing a target polymer and including at least one of a to-be-measured material or a to-be-measured product;
inputting the near-infrared spectrum signal of the to-be-measured object into a trained glass transition temperature measurement model, where the glass transition temperature measurement model is obtained by training a training sample set that includes a plurality of training samples, the plurality of training samples include a plurality of near-infrared spectrum signals obtained by performing a near-infrared spectrum measurement on a plurality of standard samples under a plurality of preset conditions in the target environment and also include a plurality of glass transition temperature labels corresponding to the plurality of near-infrared spectrum signals in a one-to-one correspondence, the plurality of preset conditions include a measurement condition designed based on uncontrollable parameters that corresponds to the target environment and that includes an environmental parameter, each of the plurality of standard samples contains the target polymer, the plurality of standard samples include standard samples having a plurality of different uncontrollable product parameters, the plurality of uncontrollable product parameters include at least one of color, raw material ratio of the target polymer, moisture content of the target polymer, a composite material parameter of the target polymer, or a composite material structural parameter of the target polymer, and each of the plurality of glass transition temperature labels is a calibrated glass transition temperature of the target polymer in one of the plurality of standard samples that corresponds to the near-infrared spectrum signal; and
determining, using the trained glass transition temperature measurement model and based on a corresponding relationship between the plurality of near-infrared spectrum signals and a plurality of glass transition temperatures, a measured glass transition temperature that corresponds to the near-infrared spectrum signal of the to-be-measured object.

In an optional embodiment, the method further includes, prior to inputting the near-infrared spectrum signal of the to-be-measured object into a trained glass transition temperature measurement model:
performing a near-infrared spectrum measurement on the plurality of standard samples under the plurality of preset conditions in the target environment to obtain the plurality of near-infrared spectrum signals of the plurality of standard samples under the plurality of preset conditions;
acquiring the calibrated glass transition temperature of the target polymer in each of the plurality of standard samples;
creating a plurality of training samples based on the plurality of near-infrared spectrum signals corresponding to each of the plurality of standard samples under the plurality of preset conditions and also based on the calibrated glass transition temperatures respectively corresponding to each of the plurality of standard samples, to obtain a training sample set;
inputting the training sample set into the glass transition temperature measurement model;
acquiring the measured glass transition temperature corresponding to each of the plurality of training samples through the glass transition temperature measurement model and based on the corresponding relationship between the plurality of near-infrared spectrum signals and the plurality of glass transition temperatures; and
iteratively training the glass transition temperature measurement model based on the measured glass transition temperature and the calibrated glass transition temperature that correspond to each of the plurality of training samples, so that the corresponding relationship between the plurality of near-infrared spectrum signals and the plurality of glass transition temperatures is adjusted to obtain the trained glass transition temperature measurement model.

In an optional embodiment, the method further includes, prior to performing a near-infrared spectrum measurement on the plurality of standard samples under the plurality of preset conditions in the target environment:
acquiring at least one boundary value of the uncontrollable parameters corresponding to the target environment;
determining a range of the uncontrollable parameters based on the at least one boundary value of the uncontrollable parameters; and
designing the plurality of preset conditions based on the value range of the uncontrollable parameters, where the plurality of preset conditions include at least one measurement condition corresponding to the at least one boundary value of the uncontrollable parameters, and at least one measurement condition corresponding to at least one intermediate value of the uncontrollable parameters.

In an optional embodiment, the method further includes, prior to performing a near-infrared spectrum measurement on the plurality of standard samples under the plurality of preset conditions in the target environment:
acquiring a boundary value of the uncontrollable product parameter corresponding to the standard samples in a target production line;
determining a range of the uncontrollable product parameters based on the at least one boundary value of the uncontrollable product parameters; and
preparing the plurality of standard samples based on the value range of the uncontrollable product parameter, where the plurality of standard samples include a standard sample corresponding to the boundary value of the uncontrollable product parameter, and a standard sample corresponding to an intermediate value of at least one uncontrollable product parameter.

In an optional embodiment, the acquiring the calibrated glass transition temperature of the target polymer in each standard sample includes:
performing measurement on each standard sample using a target glass transition temperature measurement method to obtain the calibrated glass transition temperature of the target polymer in each of the standard samples, the target glass transition temperature measurement method including at least one of DSC or DMA.

In an optional embodiment, a wavelength for the near-infrared spectrum measurement ranges from 780 nm to 2526 nm.

In an optional embodiment, the target polymer includes at least one of a thermosetting polymeric material or a thermoplastic polymeric material.

In a second aspect, some embodiments of the present application provide an apparatus for measuring a glass transition temperature. The apparatus includes:
a measuring module, configured to perform a near-infrared spectrum measurement on a to-be-measured object in a target environment to obtain a near-infrared spectrum signal of the to-be-measured object, the to-be-measured object containing a target polymer and including at least one of a to-be-measured material or a to-be-measured product;
an input module, configured to input the near-infrared spectrum signal of the to-be-measured object into a trained glass transition temperature measurement model, where the glass transition temperature measurement model is obtained by training a training sample set that includes a plurality of training samples, the plurality of training samples include a plurality of near-infrared spectrum signals obtained by performing a near-infrared spectrum measurement on a plurality of standard samples under a plurality of preset conditions in the target environment and also include a plurality of glass transition temperature labels corresponding to the plurality of near-infrared spectrum signals in a one-to-one correspondence, the plurality of preset conditions include a measurement condition designed based on uncontrollable parameters that correspond to the target environment and include an environmental parameter, each of the plurality of standard samples contains the target polymer, the plurality of standard samples include standard samples having a plurality of different uncontrollable product parameters, the plurality of uncontrollable product parameters include at least one of color, raw material ratio of the target polymer, moisture content of the target polymer, a composite material parameter of the target polymer, or a composite material structural parameter of the target polymer, and each of the plurality of glass transition temperature labels is a calibrated glass transition temperature of the target polymer in one of the plurality of standard samples that corresponds to the near-infrared spectrum signal; and
a determination module, configured to determine, using the trained glass transition temperature measurement model and based on a corresponding relationship between the plurality of near-infrared spectrum signals and a plurality of glass transition temperatures, a measured glass transition temperature that corresponds to the near-infrared spectrum signal of the to-be-measured object.

In a third aspect, some embodiments of the present application provide a device for measuring a glass transition temperature. The device includes a processor and a memory storing computer program instructions. The processor, when executing the computer program instructions, implements any one of the above methods for measuring a glass transition temperature.

In a fourth aspect, some embodiments of the present application provide a computer storage medium storing computer program instructions. When executed by a processor, the computer program instructions implement any one of the above methods for measuring a glass transition temperature.

In a fifth aspect, some embodiments of the present application provide a computer program product. When instructions stored in the computer program product are executed by a processor of an electronic device, the electronic device performs any one of the above methods for measuring a glass transition temperature.

The method, apparatus and device for measuring a glass transition temperature, the computer storage medium, and the computer program product that are provided in the embodiments of the present application can perform the near-infrared spectrum measurement on the to-be-measured object in the target environment to obtain the near-infrared spectrum signal of the to-be-measured object. The to-be-measured object includes at least one of the to-be-measured material or the to-be-measured product and contains the target polymer. Subsequently, the near-infrared spectrum signal of the to-be-measured object is input into the trained glass transition temperature measurement model. The glass transition temperature measurement model is obtained by training the training sample set that includes the plurality of training samples. The plurality of training samples include the plurality of near-infrared spectrum signals obtained by performing the near-infrared spectrum measurement on the plurality of standard samples under the plurality of preset conditions in the target environment, and also include the plurality of glass transition temperature labels corresponding to the plurality of near-infrared spectrum signals in a one-to-one correspondence. All the near-infrared spectrum signals in the training sample set are determined in the target environment, and thus a corresponding target environment for a relatively fixed and controllable production environment can be designed so that the glass transition temperature measurement model can be applicable to the specific production environment. The plurality of preset conditions include the measurement condition designed based on the uncontrollable parameter corresponding to the target environment, and the uncontrollable parameter includes the environmental parameter. Each of the plurality of standard samples contains the target polymer, and the plurality of standard samples include standard samples having the plurality of different uncontrollable product parameters. The plurality of uncontrollable product parameters include at least one of color, raw material ratio of the target polymer, moisture content of the target polymer, a composite material parameter of the target polymer, or a composite material structural parameter of the target polymer. The glass transition temperature label is the calibrated glass transition temperature of the target polymer in the standard sample that corresponds to the near-infrared spectrum signal. Thus, the training sample set contains a more comprehensive range of training samples that can cover potential results at the glass transition temperature measurement site when facing the plurality of uncontrollable measurement environment factors and the plurality of uncontrollable product factors in the industrial environment. Then, the calibrated glass transition temperature of the target polymer in the standard sample serves as the glass transition temperature label of the training sample. Consequently, the trained glass transition temperature measurement model can accurately determine the glass transition temperature of the target polymer in the to-be-measured object based on the near-infrared spectrum signals collected at the production site. In this way, a portable near-infrared spectrometer can be used as a measurement tool for non-destructive detection in the industrial production environment, thereby achieving both high convenience and precision in measurement of the glass transition temperature. The embodiment of the present application uses the trained glass transition temperature measurement model to determine the measured glass transition temperature corresponding to the near-infrared spectrum signal of the to-be-measured object, based on the corresponding relationship between the near-infrared spectrum signal and the glass transition temperature. In this way, rapid and accurate measurement of the glass transition temperature across diverse industrial applications can be achieved, thereby enhancing production efficiency and product quality management capabilities.

### BRIEF DESCRIPTION OF THE DRAWINGS

To more clearly illustrate the technical solutions of the embodiments of the present application, the drawings required for the embodiments of the present application will be briefly described below. For those skilled in the art, other drawings can also be obtained based on these drawings without creative efforts.
Fig. 1 is a flowchart of a glass transition temperature measuring method provided by an embodiment of the present application;
Fig. 2 is a flowchart of a glass transition temperature measuring method provided by another embodiment of the present application;
Fig. 3 is a structural schematic diagram of an apparatus for measuring a glass transition temperature provided by another embodiment of the present application; and
Fig. 4 is a structural schematic diagram of a device for measuring a glass transition temperature provided by another embodiment of the present application.

### DETAILED DESCRIPTION

The following will describe in detail the features and exemplary embodiments of various aspects of the present application. To make the objectives, technical solutions, and advantages of the present application clearer and more understandable, the present application will be further described in detail below with reference to the accompanying drawings and specific embodiments. It should be understood that the specific embodiments described herein are intended only to explain the present application and not to limit it. For those skilled in the art, the present application can be implemented without some of the details described herein. The following description of the embodiments is intended solely to provide a better understanding of the present application by illustrating examples thereof.

It should be noted that, throughout this document, relational terms such as "first" and "second" are used merely to distinguish one entity or operation from another, without necessarily requiring or implying any such actual relationship or sequence between the entities or operations. Moreover, the terms "comprising," "including," or any other variant thereof are intended to encompass non-exclusive inclusion, such that a process, method, article, or device including a series of elements not only includes those elements but also includes other elements not explicitly listed, or elements inherent to such a process, method, article, or device. Without further limitation, the elements defined by the phrase "including..." do not preclude additional identical elements in the process, method, article, or device that includes said elements.

Currently, the commonly used methods for measuring a glass transition temperature in industrial production include DSC and DMA. Although both DSC and DMA can provide relatively accurate results, they need to be performed in laboratory environments, which are time-consuming operations and require data analysis performed by specialized technicians. In modern factories that pursue efficient production, rapid quality inspection, and cost control, traditional measuring methods struggle to meet production demands.

Near-infrared spectrum (NIR) technology is now widely used as an efficient and non-destructive quality inspection tool in the food, chemical, and pharmaceutical industries. NIR occupies the spectral region between visible light and mid-infrared light, with a wavelength range of 780 nm to 2526 nm and a wavenumber range of 12500 cm⁻¹ to 4000 cm⁻¹. NIR belongs to the molecular vibrational spectrum that originates from the anharmonic vibrations of covalent chemical bonds and corresponds to the overtones and combination bands of molecular vibration. The NIR signals of the polymer cover overtone and combination bands of vibrations of various molecular groups, such as C-H, O-H, and N-H. Subtle changes in these bands directly reflect microscopic changes in the internal structure and the physical state of the polymer. When the composition of a sample changes, its NIR signals also change accordingly.

Related art involves exploration of the feasibility of using NIR to measure the glass transition temperature of epoxy resins. Specifically, it involves utilizing the near-infrared light within the wavelength range of 830 nm to 2630 nm to measure the glass transition temperature of epoxy resins with a glass transition temperature ranging from 45°C to 98°C. However, the accuracy of this approach is low and not yet sufficient to meet industrial-grade requirements.

Related art also involves using near-infrared light within a wavelength range from 2000 nm to 2450 nm for near-infrared spectrum analysis and measuring the glass transition temperature of the epoxy prepregs with a glass transition temperature ranging from 25°C to 42°C. However, this approach has limited applicability, being only suitable for epoxy resin prepregs and within a narrow temperature range.

Furthermore, both of the aforementioned approaches share common limitations: firstly, they only consider laboratory conditions, making it difficult for either to meet the precision requirements of industrial applications in complex real-world factory settings; secondly, they rely on near-infrared spectrometers designed for laboratory environments, with the entire set of equipment being cumbersome and unsuitable for use in industrial production sites.

In summary, although preliminary exploratory research has been conducted in laboratories on using NIR technology to measure the glass transition temperature in the related art, the related art remains unsuitable for industrial application requirements. The precision, applicability, and convenience of NIR technology in measuring the glass transition temperature in industrial application scenarios all need to be improved.

To address these issues, the inventor, through deep deliberation, ingeniously proposes a method, an apparatus, and a device for measuring a glass transition temperature, a computer storage medium, and a computer program product.

With reference to the accompanying drawings, the following describes the glass transition temperature measuring method provided by some embodiments of the present application through specific embodiments and their application scenarios. The glass transition temperature measuring method provided by the embodiments of the present application can be implemented by an apparatus for measuring a glass transition temperature or some specific modules configured to execute the glass transition temperature measuring method in the apparatus. The embodiments of the present application take the apparatus for measuring a glass transition temperature implementing the glass transition temperature measuring method as an example to specifically describe the glass transition temperature measuring method.

Additionally, it should be noted that in the glass transition temperature measuring method provided in the embodiments of the present application, after the near-infrared spectrum signal of the to-be-measured object is obtained by performing the near-infrared spectrum measurement on the to-be-measured object in the target environment, a measured glass transition temperature corresponding to the near-infrared spectrum signal needs to be determined using a glass transition temperature measurement model. Therefore, prior to the determining the measured glass transition temperature corresponding to the near-infrared spectrum signal using the glass transition temperature measurement model, the glass transition temperature measurement model needs to be trained first. The following describes a specific implementation method for training the glass transition temperature measurement model used in the method for measuring the glass transition temperature provided by the embodiments of the present application.

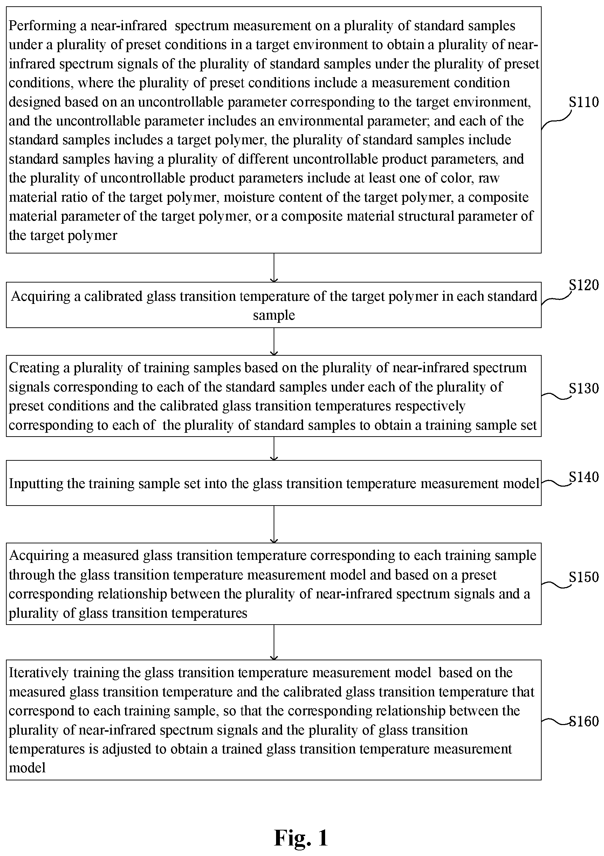

Fig. 1 is a flowchart of a glass transition temperature measuring method provided by an embodiment of the present application, specifically, a flowchart of a method for training the glass transition temperature measurement model used in the glass transition temperature measuring method provided by the embodiment of the present application.

As shown in Fig. 1, the method for training the glass transition temperature measurement model used in the glass transition temperature measuring method provided by the embodiment of the present application can include steps S110 to S160.

In S110, a near-infrared spectrum measurement is performed on a plurality of standard samples under a plurality of preset conditions in a target environment to obtain a plurality of near-infrared spectrum signals of the plurality of standard samples under the plurality of preset conditions. The plurality of preset conditions include a measurement condition designed based on an uncontrollable parameter corresponding to the target environment, and the uncontrollable parameter includes an environmental parameter. Each standard sample includes a target polymer. The plurality of standard samples include standard samples having a plurality of different uncontrollable product parameters. The plurality of uncontrollable product parameters include at least one of the following: color, raw material ratio of the target polymer, moisture content of the target polymer, a composite material parameter of the target polymer, or a composite material structural parameter of the target polymer.

Step S110 involves distinguishing parameters that affect the measurement accuracy of the glass transition temperature in actual factory environments into controllable and uncontrollable parameters. When the controllable parameters are fixed, comprehensive and representative measurement samples and measurement environments are designed for the uncontrollable parameters. Specifically, in step S110, the target environment can be a fixed measurement environment designed based on the controllable parameters at an industrial production site. For example, the target environment can be a factory setting where the background for the measurement is controlled to be fixed. The plurality of preset conditions can be a plurality of measurement environments designed based on the uncontrollable environmental parameters at the industrial production site. For example, the uncontrollable parameter corresponding to the target environment may include uncontrollable environmental parameters in a factory setting, such as, but not limited to, ambient temperature, relative humidity, and one or more environmental pollutants potentially present in the factory environment. The plurality of standard samples can be measurement samples designed with fixed product structures but varying uncontrollable product parameters based on the controllable structural parameter and the uncontrollable product parameter of at least one of a material or a product. For example, they may be specially fabricated measurement samples based on the surface structure of the product. Among the uncontrollable product parameters, the composite material parameters of the target polymer may include, for example, a type of another material compounded with the target polymer in the composite material, including but not limited to fabric types and core material types. The composite material structural parameters may include, but are not limited to, the relative orientation between fabrics and the surface morphological structure of the core material. It should be understood that the above controllable and uncontrollable parameters are not fixed and can be adjusted based on actual needs. For instance, some industrial production environments impose strict requirements on ambient temperature and relative humidity, and then the ambient temperature and the relative humidity can also serve as controllable parameters.

It should be understood that the above target environment can include a fixed measurement environment designed for the same industrial production site. The aforementioned multiple standard samples may include measurement samples designed for the same material or product with different uncontrollable product parameters. When training a glass transition temperature measurement model applicable to multiple industrial production sites and/or various materials and/or products, it is possible to distinguish, for each material and/or product at each industrial production site, the controllable parameters from the uncontrollable parameters. When the controllable parameters are fixed, comprehensive and representative measurement samples and measurement environments are designed for the uncontrollable parameters, and corresponding near-infrared spectrum signals are collected.

In S120, the calibrated glass transition temperature of the target polymer in each of the standard samples is acquired.

In step S120, the calibrated glass transition temperature may include the glass transition temperature measured by a glass transition temperature measurement method having relatively high precision. The calibrated glass transition temperature has high precision and can be regarded as the actual glass transition temperature of the target polymer in the standard sample.

In S130, a plurality of training samples are created based on the plurality of near-infrared spectrum signals corresponding to each of the standard samples under each of the preset conditions and the calibrated glass transition temperatures corresponding to each of the standard samples to obtain a training sample set.

In step S130, the calibrated glass transition temperature corresponding to the standard sample can serve as a glass transition temperature label to evaluate the effectiveness of model training.

In S140, the training sample set is input into the glass transition temperature measurement model.

In step S140, the glass transition temperature measurement model may include a correction prediction model constructed through mathematical algorithms, such as a correction prediction model constructed based on partial least squares regression, principal component regression, multiple linear regression, or neural network algorithms, which are not particularly limited in the present application.

In S150, a measured glass transition temperature corresponding to each of the training samples is acquired through the glass transition temperature measurement model based on a preset corresponding relationship between the near-infrared spectrum signals and the glass transition temperatures.

In S160, the glass transition temperature measurement model is iteratively trained based on the measured glass transition temperature and the calibrated glass transition temperature that correspond to each of the training samples, so that the corresponding relationship between the plurality of near-infrared spectrum signals and the plurality of glass transition temperatures is adjusted to obtain a trained glass transition temperature measurement model.

In step S160, the glass transition temperature measurement model is iteratively trained based on the measured glass transition temperature and the calibrated glass transition temperature that correspond to each of the training samples until a preset training termination condition is satisfied, thereby obtaining the trained glass transition temperature measurement model. This termination condition may be, for example, reaching a preset iteration times or the performance of the model meeting a preset requirement, which are not limited herein. For example, the measured glass transition temperature and the calibrated glass transition temperature of each of the training samples can be used to calculate a deviation function value of the glass transition temperature measurement model. When the deviation function value is less than a preset threshold, the trained glass transition temperature measurement model is obtained.

According to the above embodiment, the near-infrared spectrum measurement is performed on the plurality of standard samples under the plurality of preset conditions in the target environment to obtain the near-infrared spectrum signal of each of the plurality of standard samples under each of the plurality of preset conditions. The plurality of preset conditions include the measurement condition designed based on the uncontrollable parameter corresponding to the target environment, and the uncontrollable parameter includes the environmental parameter. Each of the plurality of standard samples contains the target polymer. The plurality of standard samples include the plurality of standard samples that have different uncontrollable product parameters. The uncontrollable product parameters include at least one of color, raw material ratio of the target polymer, moisture content of the target polymer, a composite material parameter of the target polymer, or a composite material structural parameter of the target polymer. Thus, for the relatively fixed controllable production site environment, a corresponding target environment can be designed; for uncontrollable product parameters, the plurality of standard samples with different uncontrollable product parameters can be designed; and for uncontrollable measurement environment factors, the plurality of preset conditions can be designed. In this way, the near-infrared spectrum signals collected for each of the standard samples under all of the preset conditions are relatively comprehensive, which can cover potential results at the glass transition temperature measurement site in the industrial environment.

Subsequently, the calibrated glass transition temperature of the target polymer in each of the standard samples can be obtained. The calibrated glass transition temperature exhibits high accuracy and can be regarded as the actual glass transition temperature of the target polymer in the standard sample. Next, the training samples are created based on the near-infrared spectrum signal corresponding to each of the standard samples under each of the preset conditions and the calibrated glass transition temperature corresponding to each of the standard samples to obtain the training sample set. The training sample set created in this way includes a rich variety of training samples with high representativeness. Subsequently, the training sample set is then input into the glass transition temperature measurement model. The measured glass transition temperature corresponding to each of the training samples is obtained through the glass transition temperature measurement model based on the preset corresponding relationship between the near-infrared spectrum signals and the glass transition temperatures. The glass transition temperature measurement model is iteratively trained based on the measured glass transition temperature and the calibrated glass transition temperature that correspond to each of the training samples, so that the corresponding relationship between the near-infrared spectrum signals and the glass transition temperatures is adjusted to obtain the trained glass transition temperature measurement model through which the glass transition temperature of the target polymer of the to-be-measured object is determined. In this way, the trained glass transition temperature measurement model can accurately determine the glass transition temperature of the target polymer based on the near-infrared spectrum signals collected in the production site.

The glass transition temperature measurement model trained based on the above implementation method can achieve high accuracy at an industrial production site. In this way, a portable near-infrared spectrometer (e.g., a handheld NIR spectrometer) can be used as a measurement tool to non-destructively, rapidly, and accurately measure the glass transition temperatures of raw materials and products in industrial applications. Consequently, the method for measuring the glass transition temperature incorporating the glass transition temperature measurement model has high applicability and can be applied to the production and maintenance of components and products in fields such as aerospace, wind power, energy storage, marine, and automotive industries. The applicable raw materials may include thermosetting and thermoplastic polymeric materials, as well as composite materials having these materials as a matrix. The applicable products may include, but are not limited to, composite components used in aircraft, wind turbine blades, energy storage tanks, automotive parts, coatings, adhesives, and structural adhesives.

In an embodiment, prior to performing a near-infrared spectrum measurement on a plurality of standard samples under a plurality of preset conditions in a target environment, the method may include:
acquiring at least one boundary value of the uncontrollable parameter corresponding to the target environment;
determining a value range of the uncontrollable parameter based on the at least one boundary value of the uncontrollable parameter; and
designing the plurality of preset conditions based on the value range of the uncontrollable parameter. The plurality of preset conditions include at least one measurement condition corresponding to the at least one boundary value of the uncontrollable parameter and at least one measurement condition corresponding to at least one intermediate value of at least one uncontrollable parameter.

In the above embodiment, the boundary values can include the extreme value that the uncontrollable parameter can reach in the target environment or the extreme value that the uncontrollable parameter may potentially reach. The value range of the uncontrollable parameter can represent a selectable range of the uncontrollable parameter. Designing the plurality of preset conditions based on the value range of the uncontrollable parameter can include designing a measurement condition corresponding to the at least one boundary value of the uncontrollable parameter and the measurement condition corresponding to at least one intermediate value of at least one uncontrollable parameter. In some examples, the uncontrollable parameter may include multiple environmental parameters, which can be combined based on their respective value ranges so that the designed preset conditions can comprehensively cover as many variations as possible in the actual production environment.

According to the above embodiment, the boundary value of the uncontrollable parameter in the target environment is acquired, the value range of the uncontrollable parameter is determined, and multiple preset conditions are then designed based on the value range of the uncontrollable parameter. In this way, the multiple preset conditions can cover as many variations as possible in the actual production environment, thereby enhancing the comprehensiveness and representativeness of the training samples. Consequently, the glass transition temperature measurement model maintains high accuracy even when faced with variable uncontrollable environmental parameters in the target environment, thereby improving the precision and flexibility of the measurement of the glass transition temperature.

In an embodiment, prior to performing a near-infrared spectrum measurement on a plurality of standard samples under a plurality of preset conditions in a target environment, the method can further include:
acquiring a boundary value of the uncontrollable product parameter corresponding to the standard sample in a target production line;
determining a value range of the uncontrollable product parameter based on the boundary value of the uncontrollable product parameter; and
preparing the plurality of standard samples based on the value range of the uncontrollable product parameter, where the plurality of standard samples include a standard sample corresponding to the boundary value of the uncontrollable product parameter and a standard sample corresponding to an intermediate value of the uncontrollable product parameter.

In the above embodiment, the boundary value can include the extreme value that the uncontrollable product parameter corresponding to the standard sample in the target production line can reach or the extreme value that the uncontrollable product parameter may potentially reach. The value range of the uncontrollable product parameter can represent a selectable range of the uncontrollable parameter. Preparing the plurality of standard samples based on the value range of the uncontrollable product parameters may include preparing the standard sample corresponding to the boundary value of the uncontrollable product parameter and the standard sample corresponding to the intermediate value of at least one uncontrollable product parameter. For example, taking the color of the target polymer as an example, for natural color differences existing between different batches, it is necessary to design a sample set with a color gradient, ranging from lightest to darkest, with a span that exceeds the color range used in actual production. In this way, the impact caused by the uncontrollable variables can be effectively corrected by means of chemical quantitative analysis to address minor variations between raw materials or product batches. In some examples, there may be two or more uncontrollable product parameters, and the uncontrollable product parameters can be combined based on their respective value ranges so that the prepared multiple standard samples can cover as many variations as possible in the actual production environment.

According to the above embodiment, the boundary value of the uncontrollable product parameter corresponding to the standard sample in the target production line is acquired, the value range of the uncontrollable product parameter is determined, and then multiple standard samples are prepared based on the value range of the uncontrollable product parameter. In this way, the multiple standard samples can cover as many variations as possible in the actual production environment, thereby enhancing the comprehensiveness and representativeness of the training samples. Consequently, the glass transition temperature measurement model maintains high accuracy when faced with variable uncontrollable product parameters in industrial production, thereby enhancing the precision and flexibility of the measurement of the glass transition temperature.

In an embodiment, the acquiring the calibrated glass transition temperature of the target polymer in each of the plurality of standard samples can specifically include:
performing measurement on each of the plurality of standard samples using a target glass transition temperature measurement method to obtain the calibrated glass transition temperature of the target polymer in each of the plurality of standard samples, where the target glass transition temperature measurement method includes at least one of DSC or DMA.

According to the above embodiment, the calibrated glass transition temperature of the target polymer in each of the standard samples is determined using at least one of DSC or DMA. Both the DSC and the DMA exhibit high precision, with the calibrated glass transition temperatures measured by the DSC and the DMA closely approximating the actual glass transition temperature of the target polymer in the standard samples. Consequently, taking the calibrated glass transition temperature as the label of the training sample enhances the precision of the glass transition temperature measurement model.

In an embodiment, performing a near-infrared spectrum measurement on a plurality of standard samples under a plurality of preset conditions in a target environment to obtain near-infrared spectrum signals of each of the standard samples under each of the preset conditions can specifically include:
performing the near-infrared spectrum measurement on the plurality of standard samples under the plurality of preset conditions in the target environment to obtain original near-infrared spectrum signals of each of the standard samples under each of the preset conditions; and
performing preprocessing on the original near-infrared spectrum signals of each of the standard samples under each of the preset conditions to obtain the near-infrared spectrum signals of each of the standard samples under each of the preset conditions.

The aforementioned preprocessing can be achieved using known preprocessing methods in the field, such as, but not limited to, at least one of the following: applying smoothing techniques to remove random noise, performing normalization operations to standardize data distribution, or performing first-order derivative transformation and second-order derivative transformation to reveal potential spectral features.

According to the above embodiment, preprocessing the original near-infrared spectrum signals facilitates the extraction of spectral features exhibited by the target polymer system as its glass transition temperature varies under the production environment. In this way, it is beneficial for the glass transition temperature measurement model to accurately identify the potential relationship between the glass transition temperatures and the near-infrared spectrum signals, thereby enhancing the accuracy of the glass transition temperature measurement.

The embodiments of the present application do not limit the wavelength for the near-infrared spectrum measurement. According to the embodiments of the present application, the glass transition temperature measurement model employed in the method for measuring the glass transition temperature has high precision and applicability, with a broad scope of applicability. Consequently, depending on the chemical structure of the target polymer, the wavelength range for the near-infrared spectrum measurement can be any portion between 780 nm and 2526 nm. For example, it can be 780 nm to 2526 nm, 780 nm to 2400 nm, 780 nm to 2200 nm, 780 nm to 2000 nm, 780 nm to 1800 nm, 780 nm to 1600 nm, 780 nm to 1200 nm, 780 nm to 1000 nm, 800 nm to 2526 nm, 800 nm to 2300 nm, 800 nm to 2100 nm, 800 nm to 1900 nm, 800 nm to 1500 nm, and the like.

In this way, different measurement wavelengths can be selected for different target polymers, ensuring relatively highly accurate measurement results for the glass transition temperature of each of the different target polymers.

In an embodiment, after iteratively training the glass transition temperature measurement model based on the measured glass transition temperature and the calibrated glass transition temperature that correspond to each of the plurality of training samples, the method can further include:
validating the performance of the glass transition temperature measurement model using actual industrial material or an actual product that corresponds to the standard sample, so as to adjust model parameters of the glass transition temperature measurement model.

In this way, the glass transition temperature measurement model can be optimized and corrected using at least one of the actual industrial materials or the actual products, thereby further enhancing the accuracy of the model.

In an embodiment, the wavelength range for the near-infrared spectrum measurement may be 950 nm to 1650 nm. This facilitates achieving high precision, broad applicability, and low instrument production costs.

In an embodiment, the target polymer may include at least one of a thermosetting polymeric material or a thermoplastic polymeric material.

Exemplarily, the thermosetting polymeric material may include epoxy resin, unsaturated polyester, polyurethane, and their modified, block-copolymerized, hybridized, or blended materials and systems. Exemplarily, the thermoplastic polymeric material may include free-radically polymerized polyolefins and their modified, block-copolymerized, hybridized, or blended polyolefin materials and systems.

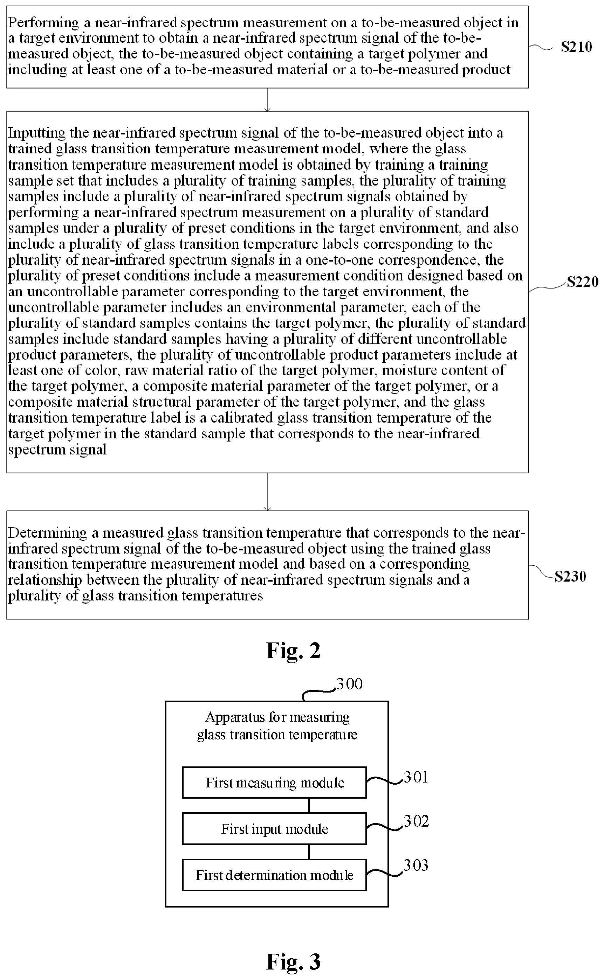

The following describes in detail the method for measuring the glass transition temperature provided by some embodiments of the present application with reference to Fig. 2.

Fig. 2 is a flowchart of a glass transition temperature measuring method provided by an embodiment of the present application. As shown in Fig. 2, the method for measuring the glass transition temperature specifically includes the following steps S210 to S230.

In S210, a near-infrared spectrum measurement is performed on a to-be-measured object in a target environment to obtain a near-infrared spectrum signal of the to-be-measured object, the to-be-measured object containing a target polymer and including at least one of a to-be-measured material or a to-be-measured product.

In step S210, the to-be-measured object may include a surface structure or a raw material of a product.

In S220, the near-infrared spectrum signal of the to-be-measured object is input into a trained glass transition temperature measurement model. The glass transition temperature measurement model is obtained by training a training sample set that includes a plurality of training samples. The plurality of training samples include a plurality of near-infrared spectrum signals obtained by performing a near-infrared spectrum measurement on a plurality of standard samples under a plurality of preset conditions in the target environment, and also include a plurality of glass transition temperature labels corresponding to the plurality of near-infrared spectrum signals in a one-to-one correspondence. The plurality of preset conditions include a measurement condition designed based on an uncontrollable parameter corresponding to the target environment, and the uncontrollable parameter includes an environmental parameter. Each of the plurality of standard samples contains the target polymer. The plurality of standard samples include standard samples having a plurality of different uncontrollable product parameters. The plurality of uncontrollable product parameters include at least one of color, raw material ratio of the target polymer, moisture content of the target polymer, a composite material parameter of the target polymer, or a composite material structural parameter of the target polymer. The glass transition temperature label is a calibrated glass transition temperature of the target polymer in the standard sample that corresponds to the near-infrared spectrum signal.

In S230, a measured glass transition temperature that corresponds to the near-infrared spectrum signal of the to-be-measured object is determined using the trained glass transition temperature measurement model and based on a corresponding relationship between the plurality of near-infrared spectrum signals and a plurality of glass transition temperatures.

The glass transition temperature measuring method that is provided in the embodiment of the present application can perform the near-infrared spectrum measurement on the to-be-measured object in the target environment to obtain the near-infrared spectrum signal of the to-be-measured object. The to-be-measured object includes at least one of the to-be-measured material or the to-be-measured product and contains the target polymer. Subsequently, the near-infrared spectrum signal of the to-be-measured object is input into the trained glass transition temperature measurement model. The glass transition temperature measurement model is obtained by training the training sample set that includes the plurality of training samples. The plurality of training samples include the plurality of near-infrared spectrum signals obtained by performing the near-infrared spectrum measurement on the plurality of standard samples under the plurality of preset conditions in the target environment, and also include the plurality of glass transition temperature labels corresponding to the plurality of near-infrared spectrum signals in a one-to-one correspondence. All the near-infrared spectrum signals in the training sample set are determined in the target environment, and thus a corresponding target environment for a relatively fixed and controllable production environment can be designed so that the glass transition temperature measurement model can be applicable to the specific production environment. The plurality of preset conditions include the measurement condition designed based on the uncontrollable parameter corresponding to the target environment, and the uncontrollable parameter includes the environmental parameter. Each of the plurality of standard samples contains the target polymer, and the plurality of standard samples include standard samples having the plurality of different uncontrollable product parameters. The plurality of uncontrollable product parameters include at least one of color, raw material ratio of the target polymer, moisture content of the target polymer, a composite material parameter of the target polymer, or a composite material structural parameter of the target polymer. The glass transition temperature label is the calibrated glass transition temperature of the target polymer in the standard sample that corresponds to the near-infrared spectrum signal. Thus, the training sample set contains a more comprehensive range of training samples that can cover potential results at the glass transition temperature measurement site when facing the plurality of uncontrollable measurement environment factors and the plurality of uncontrollable product factors in the industrial environment. Then, the calibrated glass transition temperature of the target polymer in the standard sample serves as the glass transition temperature label of the training sample. Consequently, the trained glass transition temperature measurement model can accurately determine the glass transition temperature of the target polymer in the to-be-measured object based on the near-infrared spectrum signals collected at the production site. In this way, a portable near-infrared spectrometer can be used as a measurement tool for non-destructive detection in the industrial production environment, thereby achieving both high convenience and precision in measurement of the glass transition temperature. The embodiment of the present application uses the trained glass transition temperature measurement model to determine the measured glass transition temperature corresponding to the near-infrared spectrum signal of the to-be-measured object, based on the corresponding relationship between the near-infrared spectrum signal and the glass transition temperature. In this way, rapid and accurate measurement of the glass transition temperature across diverse industrial applications can be achieved, thereby enhancing production efficiency and product quality management capabilities.

To better describe the overall scheme, a specific example based on the above embodiments is provided to illustrate the method for measuring the glass transition temperature of the present application. A detailed explanation follows. It should be noted that the following example is provided solely for illustrative purposes and does not constitute a limitation on the embodiments of the present application.

As an example, the method for measuring the glass transition temperature can include the following steps S310 to S360.

In S310, parameters affecting the accuracy of the measurement of the glass transition temperature in a factory environment are determined.

In step S310, the parameters affecting the accuracy of the measurement of the glass transition temperature may include parameters affecting the collection of the near-infrared spectrum signal, particularly those affecting the absorption characteristics and signal intensity feedback of the near-infrared spectrum. For example, they may include environmental parameters, parameters intrinsic to the polymer itself, composite material parameters of the polymer, and composite material structural parameters. The composite material parameters of the polymer may include a type of the material within a depth range that the near-infrared light can penetrate during measurement. The composite material structural parameters may include a layup sequence and an interfacial structure of the composite material. Exemplarily, the environmental factors may include ambient temperature, relative humidity, and environmental pollutants. The parameters intrinsic to the polymer itself may include at least one of color, a raw material ratio (e.g., a resin-to-curing agent mix ratio) of the polymer, and a moisture content of the polymer. The composite material parameters of the polymer and composite material structural parameters can be determined by the actual conditions of the product. For example, the composite material parameters of the polymer may include, in addition to a polymer matrix material, the type of the composite material within the depth range that the near-infrared light can penetrate, such as, but not limited to, a reinforcing material compounded with the polymer matrix material (such as glass fiber, carbon fiber, aramid fiber, boron fiber, silicon carbide fiber, and natural fiber). For the same type of fiber, it can be categorized into different kinds of fabrics, such as: normal modulus uniaxial fabrics, normal modulus biaxial fabrics, normal modulus triaxial fabrics, high modulus uniaxial fabrics, high modulus biaxial fabrics, high modulus triaxial fabrics, ultra-high modulus uniaxial fabrics, ultra-high modulus biaxial fabrics, ultra-high modulus triaxial fabrics. Exemplarily, the type of the material within the depth range that the near-infrared light can penetrate may also include foams for weight reduction and stiffness enhancement, such as polyethylene terephthalate (PET), polyvinyl chloride (PVC), polyurethane (PU), polystyrene (PS), polymethacrylimide (PMI), acrylonitrile-styrene foam, polyethylene foam, balsa wood, and engineered foam. The composite material structural parameters may include layup structures of various different combinations of composite materials, the number of fabric layers used, and angles between fabrics. The composite material structural parameters may further include the surface structure of the composite material, such as a surface structure of foam and a surface structure of balsa wood (including but not limited to grooved structures, and the structure and distribution of the reinforcement materials of the engineered foam).

In S320, the controllable parameters are fixed, and the training sample set is designed for the uncontrollable parameters.

In step S320, fixing the controllable parameters may include fixing measurement environment and fixing the measured object. For example, the fixing the measurement environment may include fixing the measurement environment to a specific factory environment and controlling the measured background to be a fixed background. The fixing the measured object may include fixing the measured object as a particular type of product or raw material and fixing the specifications of the measured object. The fixing the controllable parameters may specifically refer to simplifying the system of influencing factors when production processes permit. For example, in the case of reinforced composite materials, taking the outer surface material contacted by a near-infrared spectrometer as the design prototype, a sample that can be peeled from the product or the component without damage is created. When the outer surface of the product consists of reinforced composite materials, a thickness of the sample can be adjusted based on the number of fabric layers, such as a single layer, double layers, or multiple layers, which depends on the impact of fabric thickness on the precision of the measurement of the glass transition temperature and the balance between accuracy and operational convenience of the measurement of the glass transition temperature. For a sample with a multi-layer fabric structure, the directions of the fabric fibers can be designed to be the same or maintain a specific angle. Without affecting the accuracy of the glass transition temperature measurement, it is not necessary to strictly control the relative orientation in each of the fabric layers. After completing the manufacturing process of the product or the component, these samples can be safely detached from the production line. The glass transition temperatures of the samples can be measured by the near-infrared spectrum without affecting the spectral quality.

The designed training sample set for the uncontrollable parameters may indicate that a comprehensive training sample set is designed and constructed for those uncontrollable parameters that are difficult to predict or adjust, so as to ensure the training samples can adequately represent various scenarios in actual production environments. When designing the training sample set, in addition to covering all variations under normal production conditions, it is also necessary to expand the scope to ensure that the training samples maintain their representativeness and effectiveness even under extreme or boundary conditions. Taking the color of the polymer as an example, for natural color differences existing between different batches, it is necessary to design a sample set with a color gradient, ranging from lightest to darkest, with a span that exceeds the actual color range used in actual production. In this way, the impact caused by the uncontrollable variables can be effectively corrected by means of chemical quantitative analysis to address minor variations between raw materials or product batches. Exemplarily, designing the training sample set for the uncontrollable parameters may include: designing multiple measurement conditions corresponding to different environmental parameters for uncontrollable environmental parameters, and designing multiple standard samples with different uncontrollable product parameters for uncontrollable product parameters. The implementation methods for constructing the training sample set have been detailed above and will not be repeated herein.

In S330, the near-infrared spectrum signals and the calibrated glass transition temperatures of the training samples in the training sample set are acquired.

In step S330, the acquiring the near-infrared spectrum signals of the training samples in the training sample set may include acquiring corresponding original near-infrared spectrum signals for each of the training samples in the training sample set under the designed measurement conditions, and then preprocessing the original spectral signals, which includes, but is not limited to, applying smoothing techniques to remove random noise, performing normalization operations to standardize data distributions, and applying first-order derivative transformation and second-order derivative transformation to reveal potential spectral features. The acquiring the calibrated glass transition temperatures may include determining, using DSC or DMA, the glass transition temperature of each of the samples as the glass transition temperature label for each of the training samples.

In S340, the glass transition temperature measurement model is trained using the training sample set.

In S350, validation is performed using at least one of an actual industrial raw material or an actual product in the factory to optimize the glass transition temperature measurement model.

In S360, the near-infrared spectrum signal of the to-be-measured object is acquired and then input into the glass transition temperature measurement model to obtain the glass transition temperature of the target polymer of the to-be-measured object.

The above example identifies and controls parameters affecting the accuracy of measurement of the glass transition temperature using a systematic method, distinguishes between controllable and uncontrollable factors, and designs and constructs the training sample set, thereby enhancing the precision of measuring the glass transition temperature using the near-infrared spectrum technology in industrial applications, addressing significant measurement deviations caused by environmental, material, and product structural variations in practical production applications, and ensuring the reliability of measurement data. The method for measuring the glass transition temperature is not only applicable to various thermosetting and thermoplastic polymer materials and their composites but may also be widely applied to quality inspection of products across multiple industrial fields, including aerospace, wind power, energy storage, marine, automotive industries, and sports equipment, and offers high flexibility and universal applicability. The method for measuring the glass transition temperature implemented in the present application effectively addresses the limitations of laboratory research on near-infrared spectrum techniques for measuring the glass transition temperature in industrial applications, such as low measurement accuracy, narrow applicability, and inconvenient on-site instrument operation. It provides a fast, precise, and non-destructive technology for measuring the glass transition temperature in materials and products in relevant industrial sectors.

For the application of measuring the glass transition temperature of a shell and a web of a wind turbine blade, the method for measuring the glass transition temperature according to the embodiments of the present application is used to develop near-infrared spectroscopy measurement technology for infusion epoxy resin system A, which can non-destructively measure the glass transition temperature of the glass-fiber-reinforced composite material during the curing process. The glass transition temperature measurement model is established using the method provided in the embodiments of the present application, which is used to measure the glass transition temperatures of factory-produced shells and webs. Statistical analysis is conducted on the deviations between over 2,000 glass transition temperature data points output by the model and those measured by DSC. The standard deviation between the glass transition temperatures measured by the near-infrared spectrum and those measured by DSC is 3.0517°C. A measurement system analysis (MSA) is conducted on the method for measuring the glass transition temperature of epoxy resin A using the near-infrared spectrum technique, and the analysis results are shown in Table 1. As indicated in Table 1, the study variation (SV) for this method is 1.67%, meeting the requirements of the quality monitoring system.

**Table 1**

| Source | Standard deviation (SD) | Study Variation (6×SD) | % Study Variation (%SV) |
|---|---|---|---|
| total gauge R&R | 0.15820 | 0.9492 | 1.67 |
| repeatability | 0.14420 | 0.8652 | 1.52 |
| reproducibility | 0.6507 | 0.3904 | 0.69 |
| operator | 0.6507 | 0.3904 | 0.69 |
| Part-to-Part | 9.48359 | 56.9015 | 99.99 |
| Total Variation | 9.48491 | 56.9094 | 100.00 |
| Number of Distinct Categories=84 | | | |

Based on the same inventive concept, the present application also provides an apparatus for measuring a glass transition temperature.

As shown in Fig. 3, the apparatus 300 for measuring a glass transition temperature may include a first measuring module 301, a first input module 302, and a first determination module 303.

The first measuring module 301 is configured to perform a near-infrared spectrum measurement on a to-be-measured object in a target environment to obtain a near-infrared spectrum signal of the to-be-measured object, the to-be-measured object containing a target polymer and including at least one of a to-be-measured material or a to-be-measured product.

The first input module 302 is configured to input the near-infrared spectrum signal of the to-be-measured object into a trained glass transition temperature measurement model. The glass transition temperature measurement model is obtained by training a training sample set that includes a plurality of training samples. The plurality of training samples include a plurality of near-infrared spectrum signals obtained by performing a near-infrared spectrum measurement on a plurality of standard samples under a plurality of preset conditions in the target environment, and also include a plurality of glass transition temperature labels corresponding to the plurality of near-infrared spectrum signals in a one-to-one correspondence. The plurality of preset conditions include a measurement condition designed based on an uncontrollable parameter corresponding to the target environment, and the uncontrollable parameter includes an environmental parameter. Each of the plurality of standard samples contains the target polymer. The plurality of standard samples include standard samples having a plurality of different uncontrollable product parameters. The plurality of uncontrollable product parameters include at least one of color, raw material ratio of the target polymer, moisture content of the target polymer, a composite material parameter of the target polymer, or a composite material structural parameter of the target polymer. The glass transition temperature label is a calibrated glass transition temperature of the target polymer in the standard sample that corresponds to the near-infrared spectrum signal.

The first determination module 303 is configured to determine a measured glass transition temperature that corresponds to the near-infrared spectrum signal of the to-be-measured object using the trained glass transition temperature measurement model and based on a corresponding relationship between the plurality of near-infrared spectrum signals and a plurality of glass transition temperatures.

The apparatus for measuring a glass transition temperature that is provided in the embodiment of the present application can perform the near-infrared spectrum measurement on the to-be-measured object in the target environment to obtain the near-infrared spectrum signal of the to-be-measured object. The to-be-measured object includes at least one of the to-be-measured material or the to-be-measured product and contains the target polymer. Subsequently, the near-infrared spectrum signal of the to-be-measured object is input into the trained glass transition temperature measurement model. The glass transition temperature measurement model is obtained by training the training sample set that includes the plurality of training samples. The plurality of training samples include the plurality of near-infrared spectrum signals obtained by performing the near-infrared spectrum measurement on the plurality of standard samples under the plurality of preset conditions in the target environment, and also include the plurality of glass transition temperature labels corresponding to the plurality of near-infrared spectrum signals in a one-to-one correspondence. All the near-infrared spectrum signals in the training sample set are determined in the target environment, and thus a corresponding target environment for a relatively fixed and controllable production environment can be designed so that the glass transition temperature measurement model can be applicable to the specific production environment. The plurality of preset conditions include the measurement condition designed based on the uncontrollable parameter corresponding to the target environment, and the uncontrollable parameter includes the environmental parameter. Each of the plurality of standard samples contains the target polymer, and the plurality of standard samples include standard samples having the plurality of different uncontrollable product parameters. The plurality of uncontrollable product parameters include at least one of color, raw material ratio of the target polymer, moisture content of the target polymer, a composite material parameter of the target polymer, or a composite material structural parameter of the target polymer. The glass transition temperature label is the calibrated glass transition temperature of the target polymer in the standard sample that corresponds to the near-infrared spectrum signal. Thus, the training sample set contains a more comprehensive range of training samples that can cover potential results at the glass transition temperature measurement site when facing the plurality of uncontrollable measurement environment factors and the plurality of uncontrollable product factors in the industrial environment. Then the calibrated glass transition temperature of the target polymer in the standard sample serves as the glass transition temperature label of the training sample. Consequently, the trained glass transition temperature measurement model can accurately determine the glass transition temperature of the target polymer in the to-be-measured object based on the near-infrared spectrum signals collected at the production site. In this way, a portable near-infrared spectrometer can be used as a measurement tool for non-destructive detection in the industrial production environment, thereby achieving both high convenience and precision in measurement of the glass transition temperature. The embodiment of the present application uses the trained glass transition temperature measurement model to determine the measured glass transition temperature corresponding to the near-infrared spectrum signal of the to-be-measured object, based on the corresponding relationship between the near-infrared spectrum signal and the glass transition temperature. In this way, rapid and accurate measurement of the glass transition temperature across diverse industrial applications can be achieved, thereby enhancing production efficiency and product quality management capabilities.

In an embodiment, the apparatus can further include:
a second measuring module, configured to, prior to inputting the near-infrared spectrum signal of the to-be-measured object into a trained glass transition temperature measurement model, perform a near-infrared spectrum measurement on the plurality of standard samples under the plurality of preset conditions in the target environment to obtain the near-infrared spectrum signal of each of the standard samples under each of the preset conditions;
a first acquisition module, configured to acquire the calibrated glass transition temperature of the target polymer in each of the standard samples;
a creation module, configured to create the plurality of training samples based on the plurality of near-infrared spectrum signals of the plurality of standard samples under the plurality of preset conditions and also based on the calibrated glass transition temperatures respectively corresponding to the plurality of standard samples, to obtain the training sample set;
a second input module, configured to input the training sample set into the glass transition temperature measurement model;
a processing module, configured to obtain a measured glass transition temperature corresponding to each of the training samples through the glass transition temperature measurement model and based on a preset corresponding relationship between the plurality of near-infrared spectrum signals and a plurality of glass transition temperatures; and
a training module, configured to iteratively train the glass transition temperature measurement model based on the measured glass transition temperature and the calibrated glass transition temperature that correspond to each of the training samples, so that the corresponding relationship between the plurality of near-infrared spectrum signals and the plurality of glass transition temperatures is adjusted to obtain a trained glass transition temperature measurement model.

In an embodiment, the apparatus can further include:
a second acquisition module, configured to, prior to performing a near-infrared spectrum measurement on a plurality of standard samples under a plurality of preset conditions in a target environment, configured to acquire at least one boundary value of the uncontrollable parameters corresponding to the target environment;
a second determination module, configured to determine a value range of the uncontrollable parameters based on the at least one boundary value of the uncontrollable parameters; and
a design module, configured to design the plurality of preset conditions based on the value range of the uncontrollable parameters. The plurality of preset conditions include at least one measurement condition corresponding to the at least one boundary value of the uncontrollable parameters, and at least one measurement condition corresponding to at least one intermediate value of the uncontrollable parameters.

In an embodiment, the apparatus may further include:
a third acquisition module, configured to, prior to performing a near-infrared spectrum measurement on a plurality of standard samples under a plurality of preset conditions in a target environment, acquire at least one boundary value of the uncontrollable product parameters that correspond to the plurality of standard samples in a target production line;
a third determination module, configured to determine a value range of the uncontrollable product parameters based on the at least one boundary value of the uncontrollable product parameters; and
a preparation module, configured to prepare the plurality of standard samples based on the value range of the uncontrollable product parameters. The plurality of standard samples include at least one standard sample corresponding to the at least one boundary value of the uncontrollable product parameters, and at least one standard sample corresponding to at least one intermediate value of the uncontrollable product parameters.

In an embodiment, the first acquisition module, configured to acquire the calibrated glass transition temperature of the target polymer in each of the standard samples, specifically includes:
a measuring sub-module, configured to perform measurement on each of the standard samples using a target glass transition temperature measurement method to obtain the calibrated glass transition temperature of the target polymer in each of the standard samples, the target glass transition temperature measurement method including at least one of DSC or DMA.

In an embodiment, a wavelength for the near-infrared spectrum measurement ranges from 780 nm to 2526 nm.

In an embodiment, the target polymer can include at least one of a thermosetting polymeric material or a thermoplastic polymeric material.

The apparatus for measuring a glass transition temperature provided by the embodiments of the present application can implement each process realized by the method embodiments shown in Fig. 2. To avoid repetition, these processes are not repeated herein.

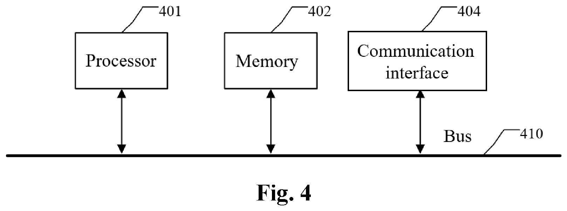

Fig. 4 is a structural schematic diagram of hardware of a device for measuring a glass transition temperature provided by some embodiments of the present application.

The device for measuring a glass transition temperature may include a processor 401 and a memory 402 storing computer program instructions.

Specifically, the processor 401 may include a central processing unit (CPU), an application-specific integrated circuit (ASIC), or one or more integrated circuits configured to implement the embodiments of the present application.

The memory 402 can include a mass storage device for data or instructions. For example, without limitation, the memory 402 can include a hard disk drive (HDD), a floppy disk drive, a flash memory, an optical disk, a magneto-optical disk, magnetic tape, or a universal serial bus (USB) drive, or a combination of two or more thereof. Where appropriate, the memory 402 can include removable or non-removable (or fixed) media. Where appropriate, the memory 402 can reside inside or outside the integrated gateway disaster recovery device. In particular embodiments, the memory 402 is a non-volatile solid-state storage.

The memory can include a read-only memory (ROM), a random access memory (RAM), disk storage media devices, optical storage media devices, flash memory devices, and electrical, optical, or other physical/tangible storage media devices. Thus, typically, the memory includes one or more tangible (non-transitory) computer-readable storage media (e.g., storage devices) encoded with software including computer-executable instructions, and when executed (e.g., by one or more processors), it is operable to perform operations described in reference to the methods according to an aspect of the present disclosure.

The processor 401 implements any method for measuring the glass transition temperature described in the above embodiments by reading and executing computer program instructions stored in the memory 402.

As an example, the device for measuring the glass transition temperature can further include a communication interface 404 and a bus 410. As shown in Fig. 4, the processor 401, the memory 402, and the communication interface 404 are connected via the bus 410 to facilitate communication among them.

The communication interface 404 is primarily used to implement communication among the various modules, apparatuses, units, and/or devices within the embodiments of the present application.

The bus 410 includes hardware, software, or both, and interconnects the components of the device for measuring a glass transition temperature. By way of example and not limitation, the bus can include an accelerated graphics port (AGP) or other graphics bus, an Extended Industry Standard Architecture (EISA) bus, a front side bus (FSB), hyper-transport (HT) interconnect, an Industry Standard Architecture (ISA) bus, InfiniBand interconnect, a low pin count (LPC) bus, a memory bus, a micro-channel architecture (MCA) bus, a peripheral component interconnect (PCI) bus, a PCI-Express (PCI-X) bus, a serial advanced technology attachment (SATA) bus, a Video Electronics Standards Association local bus (VLB), or other suitable buses, or a combination of two or more thereof. Where appropriate, the bus 410 can include one or more buses. Although specific buses are described and illustrated in the embodiments of the present application, any suitable bus or interconnect is contemplated.

The device for measuring the glass transition temperature can perform the method for measuring the glass transition temperature provided by the embodiments of the present application, thereby implementing the method for measuring the glass transition temperature and the apparatus for measuring the glass transition temperature that respectively are described with reference to Figs. 2 and 3.

Additionally, in conjunction with the data processing methods described in the above embodiments, the present application can provide a computer storage medium for implementation. The computer storage medium stores computer program instructions; when executed by a processor, the computer program instructions implement any of the methods for measuring the glass transition temperature described in the above embodiments.

The present application further provides a computer program product including a computer program. When executed by a processor, the computer program implements any of the methods for measuring the glass transition temperature described in the above embodiments.

It should be clear that the present application is not limited to the specific configurations and processing described above and illustrated in the figures. For brevity, detailed descriptions of known methods have been omitted. In the above embodiments, several specific steps are described and illustrated as examples. However, the method process of the present application is not limited to the specific steps described and illustrated. Those skilled in the art, upon understanding the gist of the present application, can make various changes, modifications, and additions, or alter the order of the steps.

The functional blocks shown in the block diagram described above can be implemented as hardware, software, firmware, or a combination thereof. When implemented as hardware, they can be, for example, electronic circuits, ASICs, appropriate firmware, plug-ins, function cards, and the like. When implemented as software, the elements of the present application are programs or code segments used to perform the required tasks. The programs or the code segments can be stored on a machine-readable medium or transmitted over a transmission medium or communication link via data signals carried by a carrier. "Machine-readable media" can include any medium capable of storing or transmitting information. Examples of machine-readable media include electronic circuits, semiconductor memory devices, a ROM, a flash memory, an erasable ROM (EROM), floppy disks, CD-ROMs, optical disks, hard disks, fiber optic media, radio frequency (RF) links, etc. The code segments can be downloaded via computer networks such as the Internet or intranets.

It should also be noted that the exemplary embodiments described herein describe certain methods or systems based on a series of steps or devices. However, the present application is not limited to the sequence of steps described above. That is, the steps can be performed in the order described in the embodiments, in a different order than described in the embodiments, or multiple steps can be performed simultaneously.

The various aspects of the present disclosure have been described with reference to flowcharts and/or block diagrams illustrating methods, apparatus (systems), and computer program products according to embodiments of the present disclosure. It should be understood that each box in the flowcharts and/or block diagrams, and combinations of boxes in the flowcharts and/or block diagrams, can be implemented by the computer program instructions. These computer program instructions can be provided to a processor of a general-purpose computer, a dedicated computer, or other programmable data processing apparatus to produce a machine, so that the instructions, when executed by the processor of the computer or other programmable data processing apparatus, enable the implementation of the functions/actions specified in one or more boxes of the flowchart and/or block diagram. Such a processor can be, but is not limited to, a general-purpose processor, a dedicated processor, a special application processor, or a field-programmable logic device. It is also understood that each block in the block diagram and/or flowchart, as well as combinations of blocks in the block diagram and/or flowchart, can also be implemented by dedicated hardware performing the specified function or action, or by a combination of dedicated hardware and computer instructions.

The foregoing describes specific embodiments of the present application. Those skilled in the art will readily appreciate that, for the sake of description and brevity, the specific operational processes of the systems, modules, and units described above can be understood by reference to the corresponding processes in the preceding method embodiments and are not repeated herein. It should be understood that the protection scope of the present application is not limited to the above. Any person skilled in the art can readily conceive various equivalent modifications or replacements within the scope of the technology disclosed herein, and such modifications or replacements should be encompassed within the protection scope of the present application.

## Claims

1. A glass transition temperature measuring method, comprising:
performing a near-infrared spectrum measurement on a to-be-measured object in a target environment to obtain a near-infrared spectrum signal of the to-be-measured object, the to-be-measured object containing a target polymer and comprising at least one of a to-be-measured material or a to-be-measured product;
inputting the near-infrared spectrum signal of the to-be-measured object into a trained glass transition temperature measurement model, wherein
the glass transition temperature measurement model is obtained by training a training sample set that comprises a plurality of training samples,
the plurality of training samples comprise a plurality of the near-infrared spectrum signals obtained by performing a near-infrared spectrum measurement on a plurality of standard samples under a plurality of preset conditions in the target environment, and a plurality of glass transition temperature labels corresponding to the plurality of near-infrared spectrum signals in a one-to-one correspondence,
the plurality of preset conditions comprise a measurement condition designed based on uncontrollable parameters corresponding to the target environment, the uncontrollable parameters comprising an environmental parameter,
each of the plurality of standard samples contains the target polymer,
the plurality of standard samples comprise standard samples having a plurality of different uncontrollable product parameters,
the plurality of uncontrollable product parameters comprise at least one of color, raw material ratio of the target polymer, moisture content of the target polymer, a composite material parameter of the target polymer, or a composite material structural parameter of the target polymer, and
each of the plurality of glass transition temperature labels is a calibrated glass transition temperature of the target polymer in a corresponding one of the plurality of standard samples that corresponds to the near-infrared spectrum signal; and
determining, using the trained glass transition temperature measurement model and based on a corresponding relationship between the plurality of near-infrared spectrum signals and a plurality of glass transition temperatures, a measured glass transition temperature that corresponds to the near-infrared spectrum signal of the to-be-measured object.

2. The method according to claim 1, further comprising, prior to the inputting the near-infrared spectrum signal of the to-be-measured object into a trained glass transition temperature measurement model:
performing a near-infrared spectrum measurement on the plurality of standard samples under the plurality of preset conditions in the target environment to obtain the plurality of near-infrared spectrum signals of the plurality of standard samples under the plurality of preset conditions;
acquiring the calibrated glass transition temperature of the target polymer in each of the plurality of standard samples;
creating the plurality of training samples based on the plurality of near-infrared spectrum signals corresponding to each of the plurality of standard samples under each of the plurality of preset conditions and the calibrated glass transition temperatures respectively corresponding to each of the plurality of standard samples, to obtain the training sample set;
inputting the training sample set into the glass transition temperature measurement model;
acquiring the measured glass transition temperature corresponding to each of the plurality of training samples through the glass transition temperature measurement model and based on the corresponding relationship between the plurality of near-infrared spectrum signals and the plurality of glass transition temperatures; and
iteratively training the glass transition temperature measurement model based on the measured glass transition temperature and the calibrated glass transition temperature that correspond to each of the plurality of training samples, so that the corresponding relationship between the plurality of near-infrared spectrum signals and the plurality of glass transition temperatures is adjusted to obtain the trained glass transition temperature measurement model.

3. The method according to claim 2, further comprising, prior to the performing a near-infrared spectrum measurement on the plurality of standard samples under the plurality of preset conditions in the target environment:
acquiring at least one boundary value of the uncontrollable parameters corresponding to the target environment;
determining a value range of the uncontrollable parameters based on the at least one boundary value of the uncontrollable parameters; and
designing the plurality of preset conditions based on the value range of the uncontrollable parameters, wherein
the plurality of preset conditions comprise:
at least one measurement condition corresponding to the at least one boundary value of the uncontrollable parameters, and
at least one measurement condition corresponding to at least one intermediate value of the uncontrollable parameters.

4. The method according to claim 2, further comprising, prior to the performing a near-infrared spectrum measurement on the plurality of standard samples under the plurality of preset conditions in the target environment:
acquiring at least one boundary value of the plurality of uncontrollable product parameters that correspond to the plurality of standard samples in a target production line;
determining a value range of the uncontrollable product parameters based on the at least one boundary value of the uncontrollable product parameters; and
preparing the plurality of standard samples based on the value range of the plurality of uncontrollable product parameters, wherein
the plurality of standard samples comprise:
at least one standard sample corresponding to the at least one boundary value of the plurality of uncontrollable product parameters, and
at least one standard sample corresponding to at least one intermediate value of the plurality of uncontrollable product parameters.

5. The method according to claim 2, wherein the acquiring the calibrated glass transition temperature of the target polymer in each of the plurality of standard samples comprises:
performing measurement on each of the plurality of standard samples using a target glass transition temperature measurement method to obtain the calibrated glass transition temperature of the target polymer in each of the plurality of standard samples, the target glass transition temperature measurement method comprising at least one of differential scanning calorimetry or dynamic mechanical analysis.

6. The method according to claim 1, wherein a wavelength for the near-infrared spectrum measurement ranges from 780 nm to 2526 nm.

7. The method according to any one of claims 1 to 6, wherein the target polymer comprises at least one of a thermosetting polymeric material or a thermoplastic polymeric material.

8. A glass transition temperature measuring method, comprising:
performing a near-infrared spectrum measurement on a plurality of standard samples under a plurality of preset conditions in a target environment to obtain a plurality of near-infrared spectrum signals of each of the plurality of standard samples under each of the plurality of preset conditions, wherein
the plurality of preset conditions comprise a measurement condition designed based on uncontrollable parameters corresponding to the target environment, the uncontrollable parameters comprising an environmental parameter;
the target environment is a fixed measurement environment designed based on controllable parameters at an industrial production site;
each of the plurality of standard samples contains a target polymer;
the plurality of standard samples comprise a plurality of standard samples that are designed based on controllable structural parameters and uncontrollable product parameters of at least one of a material or a product, have a fixed product structure, and have different uncontrollable product parameters; and
the uncontrollable product parameters comprise at least one of color, raw material ratio of the target polymer, moisture content of the target polymer, a composite material parameter of the target polymer, or a composite material structural parameter of the target polymer;
acquiring a calibrated glass transition temperature of the target polymer in each of the plurality of standard samples;
creating a plurality of training samples based on the plurality of near-infrared spectrum signals corresponding to each of the plurality of standard samples under each of the plurality of preset conditions and the calibrated glass transition temperatures respectively corresponding to each of the plurality of standard samples, to obtain a training sample set;
inputting the training sample set into a glass transition temperature measurement model;
acquiring a measured glass transition temperature corresponding to each of the plurality of training samples through the glass transition temperature measurement model and based on a preset corresponding relationship between the plurality of near-infrared spectrum signals and a plurality of glass transition temperatures; and
iteratively training the glass transition temperature measurement model based on the measured glass transition temperature and the calibrated glass transition temperature that correspond to each of the plurality of training samples, so that the corresponding relationship between the plurality of near-infrared spectrum signals and the plurality of glass transition temperatures is adjusted to obtain a trained glass transition temperature measurement model.

9. The method according to claim 8, further comprising, prior to the performing a near-infrared spectrum measurement on a plurality of standard samples under a plurality of preset conditions in a target environment:
acquiring at least one boundary value of the uncontrollable parameters corresponding to the target environment;
determining a value range of the uncontrollable parameters based on the at least one boundary value of the uncontrollable parameters; and
designing the plurality of preset conditions based on the value range of the uncontrollable parameters, wherein
the plurality of preset conditions comprise:
at least one measurement condition corresponding to the at least one boundary value of the uncontrollable parameters, and
at least one measurement condition corresponding to at least one intermediate value of the uncontrollable parameters.

10. The method according to claim 8, further comprising, prior to the performing a near-infrared spectrum measurement on a plurality of standard samples under a plurality of preset conditions in a target environment:
acquiring at least one boundary value of the plurality of uncontrollable product parameters that correspond to the plurality of standard samples in a target production line;
determining a value range of the uncontrollable product parameter based on the at least one boundary value of the uncontrollable product parameters; and
preparing the plurality of standard samples based on the value range of the plurality of uncontrollable product parameters, wherein
the plurality of standard samples comprise:
at least one standard sample corresponding to the at least one boundary value of the plurality of uncontrollable product parameters, and
at least one standard sample corresponding to at least one intermediate value of the plurality of uncontrollable product parameters.

11. The method according to claim 8, wherein the acquiring a calibrated glass transition temperature of the target polymer in each of the plurality of standard samples comprises:
performing measurement on each of the plurality of standard samples using a target glass transition temperature measurement method to obtain the calibrated glass transition temperature of the target polymer in each of the plurality of standard samples, the target glass transition temperature measurement method comprising at least one of differential scanning calorimetry or dynamic mechanical analysis.

12. The method according to claim 8, wherein after the iteratively training the glass transition temperature measurement model based on the measured glass transition temperature and the calibrated glass transition temperature that correspond to each of the plurality of training samples, so that the corresponding relationship between the plurality of near-infrared spectrum signals and the plurality of glass transition temperatures is adjusted to obtain a trained glass transition temperature measurement model, the method further comprises:
performing a near-infrared spectrum measurement on a to-be-measured object in the target environment to obtain a near-infrared spectrum signal of the to-be-measured object, the to-be-measured object containing the target polymer and comprising at least one of a to-be-measured material or a to-be-measured product;
inputting the near-infrared spectrum signal of the to-be-measured object into the trained glass transition temperature measurement model; and
determining, using the trained glass transition temperature measurement model and based on the corresponding relationship between the plurality of near-infrared spectrum signals and the plurality of glass transition temperatures, a measured glass transition temperature that corresponds to the near-infrared spectrum signal of the to-be-measured object.

13. The method according to claim 8, wherein a wavelength for the near-infrared spectrum measurement ranges from 780 nm to 2526 nm.

14. The method according to any one of claims 1 to 13, wherein the target polymer comprises at least one of a thermosetting polymeric material or a thermoplastic polymeric material.

15. An apparatus for measuring a glass transition temperature, comprising:
a measuring module, configured to perform a near-infrared spectrum measurement on a to-be-measured object in a target environment to obtain a near-infrared spectrum signal of the to-be-measured object, the to-be-measured object containing a target polymer and comprising at least one of a to-be-measured material or a to-be-measured product;
an input module, configured to input the near-infrared spectrum signal of the to-be-measured object into a trained glass transition temperature measurement model, wherein
the glass transition temperature measurement model is obtained by training a training sample set that comprises a plurality of training samples,
the plurality of training samples comprise a plurality of near-infrared spectrum signals obtained by performing a near-infrared spectrum measurement on a plurality of standard samples under a plurality of preset conditions in the target environment, and a plurality of glass transition temperature labels corresponding to the plurality of near-infrared spectrum signals in a one-to-one correspondence,
the plurality of preset conditions comprise a measurement condition designed based on uncontrollable parameters corresponding to the target environment, the uncontrollable parameters comprising an environmental parameter,
each of the plurality of standard samples contains the target polymer,
the plurality of standard samples comprise standard samples having a plurality of different uncontrollable product parameters,
the plurality of uncontrollable product parameters comprise at least one of color, raw material ratio of the target polymer, moisture content of the target polymer, a composite material parameter of the target polymer, or a composite material structural parameter of the target polymer, and
each of the plurality of glass transition temperature labels is a calibrated glass transition temperature of the target polymer in one of the plurality of standard samples that corresponds to the near-infrared spectrum signal; and
a determination module, configured to determine, using the trained glass transition temperature measurement model and based on a corresponding relationship between the plurality of near-infrared spectrum signals and a plurality of glass transition temperatures, a measured glass transition temperature that corresponds to the near-infrared spectrum signal of the to-be-measured object.

16. An apparatus for measuring a glass transition temperature, comprising:
a measuring module, configured to perform a near-infrared spectrum measurement on a plurality of standard samples under a plurality of preset conditions in a target environment to obtain a plurality of near-infrared spectrum signals of each of the plurality of standard samples under each of the plurality of preset conditions, wherein
the plurality of preset conditions comprise a measurement condition designed based on uncontrollable parameters corresponding to the target environment, the uncontrollable parameters comprising an environmental parameter,
the target environment is a fixed measurement environment designed based on controllable parameters at an industrial production site,
each of the plurality of standard samples contains a target polymer,
the plurality of standard samples comprise a plurality of standard samples that are designed based on controllable structural parameters and uncontrollable product parameters of at least one of a material or a product, have a fixed product structure, and have different uncontrollable product parameters, and
the uncontrollable product parameters comprise at least one of color, raw material ratio of the target polymer, moisture content of the target polymer, a composite material parameter of the target polymer, or a composite material structural parameter of the target polymer;
an acquisition module, configured to acquire a calibrated glass transition temperature of the target polymer in each of the plurality of standard samples;
a creation module, configured to create a plurality of training samples based on the plurality of near-infrared spectrum signals of the plurality of standard samples each under each of the plurality of preset conditions and the calibrated glass transition temperatures respectively corresponding to the plurality of standard samples, to obtain a training sample set;
an input module, configured to input the training sample set into a glass transition temperature measurement model;
a processing module, configured to obtain, through the glass transition temperature measurement model and based on a preset corresponding relationship between the plurality of near-infrared spectrum signals and a plurality of glass transition temperatures, a measured glass transition temperature corresponding to each of the plurality of training samples; and
a training module, configured to iteratively train the glass transition temperature measurement model based on the measured glass transition temperature and the calibrated glass transition temperature that correspond to each of the plurality of training samples, so that the corresponding relationship between the plurality of near-infrared spectrum signals and the plurality of glass transition temperatures is adjusted to obtain a trained glass transition temperature measurement model.

17. An electronic device, comprising:
a processor; and
a memory storing computer program instructions, which, when executed by the processor, implement the glass transition temperature measuring method according to any one of claims 1-14.

18. A computer-readable storage medium, storing computer program instructions, wherein when executed by a processor, the computer program instructions implement the glass transition temperature measuring method according to any one of claims 1-14.

19. A computer program product, wherein when instructions stored in the computer program product are executed by a processor of an electronic device, the electronic device is caused to perform the glass transition temperature measuring method according to any one of claims 1-14.
